Europäisches Patentamt

European Patent Office

Office européen des brevets

(19)

(11) Publication number: **0 319 664**
**A1**

# EUROPEAN PATENT APPLICATION

(21) Application number: 88116537.7

(51) Int. Cl.4: **A61K 37/14** , **A61K 37/18** ,
**C12P 21/06**

(22) Date of filing: 06.10.88

| | |
|---|---|
| Claims for the following Contracting States: ES + GR.<br><br>The title of the invention has been amended (Guidelines for Examination in the EPO, A-III, 7.3).<br><br>(30) Priority: 14.10.87 IT 2227687<br><br>(43) Date of publication of application: **14.06.89 Bulletin 89/24**<br><br>(84) Designated Contracting States:<br>**AT BE CH DE ES FR GB GR IT LI LU NL SE** | (71) Applicant: **ITALFARMACO S.p.A.**<br>**Viale Fulvio Testi, 330**<br>**I-20126 Milan(IT)**<br><br>(72) Inventor: **Sportoletti, Giancarlo**<br>**Viale Fulvio Testi, 330**<br>**I-20126 Milano(IT)**<br>Inventor: **Cremonesi, Pietro**<br>**Viale Fulvio Testi, 330**<br>**I-20126 Milano(IT)**<br><br>(74) Representative: **Minoja, Fabrizio**<br>**Studio Consulenza Brevettuale Via Rossini, 8**<br>**I-20122 Milano(IT)** |

(54) Soluble, iron-containing polypeptide derivatives, their preparation and pharmaceutical uses.

(57) Polypeptide derivatives containing 0.1 to 45% by weight of iron, which are highly soluble at neutral/basic pH and insoluble at acid pH, obtained by a) hydrolysis of succinylated proteins containing iron (FeSP), or b) interaction of succinylated peptides with iron salts (II or III). Said derivatives are very good iron carriers after by oral administration in mammals and show an extremely good tolerability.

EP 0 319 664 A1

# SOLUBLE POLYPEPTIDE DERIVATIVES HAVING A HIGH IRON CONTENT FOR MARTIAL THERAPY, PROCESSES FOR THE PREPARATION THEREOF AND PHARMACEUTICAL COMPOSITIONS THEREFROM

The present invention relates to polypeptide derivatives containing 0.1 to 45% by weight of iron, highly soluble at neutral or basic pH and insoluble in acid pH, which proved to be very good iron carriers after oral administration in mammals and perfectly tolerable.

The present invention also relates to the salts of the above mentioned derivatives with alkali and/or alkali metals and with pharmaceutically acceptable organic bases.

The present invention also provides the processes for the preparation of said derivatives and of the salts thereof, as well as pharmaceutical compositions for human or veterinary use, containing said derivatives and/or the salts thereof.

Iron plays a primary role in human and animal organisms, forming part of structures such as hemoglobin and different enzymes.

The pathological manifestation of the iron deficiency is hypochromic syderopenic anemia, which can result from various causes: blood losses of various origin, neoplasias, deficient absorption, etc..

Per os therapy of syderopenic anemia is carried out with inorganic or organic salts or complex derivatives and often involves undesired side-effects, such as gastro-intestinal injuries, diarrhea and vomit.

In the italian patent N. 1150213 in the Applicant's name protein derivatives were disclosed having high molecular weight, deriving from the interaction of succinylated proteins with iron III ions, the protein being of various origin, animal or vegetal, with different succinylation degrees and iron contents from 0.1 to 20% by weight, the higher range being related to solubility and biochemical characteristics which an iron carrier, to be bioavailable and free from side-effects, must show. In fact, for iron contents higher than 20% w/w, the derivatives of the above cited patent are insoluble an any pH value (basic, neutral and acid).

Moreover, the same derivatives having an iron content of about 10% or higher, when in solutions of concentrations suited for pharmaceutical purposes, show such an high viscosity as to make difficult the use thereof. Accordingly, commercially available drugs use said known compounds (hereinafter defined FeSP, i.e. Fe-Succinylated Proteins) having an iron content of 5-6% by weight.

Now, it has surprisingly been found that said derivatives, appropriately hydrolyzed by means of proteases, give compounds having an iron content even markedly higher than the above indicated limit, while showing optimum solubility and biochemical characteristics. In fact, they are soluble in basic and neutral medium, whilst they are insoluble at acid pH values. Said first characteristic is essential for an optimum iron absorption at the gastro-duodenal level (neutral-basic pH) and for the removal of intestinal injuring and diarrhea effects (related to the free iron, which under said pH conditions produces insoluble highly irritative hydrate-oxides), unsolubility in acid medium avoids the release of iron ions in stomach, which results in the absence of the above cited gastro-injuring effects. Moreover, solutions of said novel high-content iron derivatives have very low viscosities even at high concentrations.

It has also surprisingly been found that under the hydrolysis conditions used, no substantial changes occur in absolute iron content in the starting derivative subjected to the hydrolytic process, said content appearing in toto in the final product. As an example, subjecting to protein hydrolysis a derivative containing 5% w/w of iron, a product containing about 10% w/w of iron is recovered, no free neither precipitated iron being evidenced in the reaction medium, but only peptide residues.

A first process of the invention for the preparation of the polypeptide derivatives containing iron consists in treating iron succinyl protein solutions or suspensions (independently from Fe content) with pure or admixed proteases, under appropriate pH and temperature conditions, the derivative being recovered by acidification (conditions under which the enzyme or the products of self-hydrolysis thereof are soluble), followed by subsequent re-dissolutions at neutral pH and re-precipitations (for the purposes of a complete purification), while the final solid product is obtained by removing the aqueous solvent at neutral pH by evaporation under vacuum, lyophilization or spray-drying.

The same derivatives can also be prepared by interaction of iron ion with both the products from a partial enzymatic hydrolysis of succinylated proteins, and with succinylated polypeptides obtained by succinylation of peptides (deriving from primary hydrolysis of animal or vegetal proteins or from synthesis) having different succinylation degrees (10% to 100% of succinylable groups present in the native proteins or in the peptides). Therefore, the object of the present invention is provided by iron-polypeptide complexes (having an iron content from 0.1 to 45% w/w), obtained:

a) by enzymatic degradation, under appropriate pH and temperature conditions, of the iron succinyl protein (FeSP) complexes disclosed by Italian Patent n. 1.150.213; or

b) by interaction of iron ion with succinylated polypeptides obtained by hydrolysis of succinylated proteins having different succinylation degrees, said proteins being of both animal and vegetal origin; or

c) by interaction of the iron ion with succinylated polypeptides having different succinylation degrees, obtained by reacting polypeptides (from hydrolysis of animal or vegetal proteins or by synthesis) with succinic anhydride or succinoyl mono-chloride, under appropriate conditions.

Said derivatives are characterized (as evidenced by the following non-limiting examples) in that they are highly soluble in neutral or basic aqueous medium and insoluble in acid medium; they can release iron by oral administration to mammals in an higher effectiveness than the already known FeSp, at equal iron contents; and they are more suited to the preparation of pharmaceutical formulations in aqueous carrier, due to the higher solubility, the higher iron content and the lower viscosity of the resulting solutions.

EXAMPLE 1

A) Preparation of FeSp;

According to the procedure of the above mentioned patent, 1 g of succinylated casein was dissolved in 20 ml of water; 20 ml of a $FeCl_3$ aqueous solution were added (10-30 mg/ml, depending on the iron content desired to be present in the FeSP complex). The formed precipitate was dissolved at pH 7.2 and re-precipitated at pH lower than 3.5, to remove the unreacted iron excess. FeSP was dissolved in water by addition of NaOH (to pH 7.2) and liophylized. 500 mg of the resulting FeSP as the sodium salt (containing 5.2% fe w/w) were finally dissolved in 20 ml of water and pH of the solution was adjusted to 7.5 - 8.0.

B) Enzymatic degradation.

The above solution was added with trypsin at such a concentration to obtain an enzyme to substrate ratio of 1:100: specific activity of the enzyme was higher than 5.000 BAEE U/mg of protein. The hydrolysis reaction was effected at $0°-50°C$ (preferably at $25-30°C$), keeping the pH constant between 6 and 10, preferably between 7.5 and 8.5, by constant addition of 0.1 N NaOH. The reaction was continued for about 4 hours, or anyway to a reduction by about 1/10 of the starting enzymatic activity, and was completed by acidification of the solution (pH 2-3). A red-brown precipitate and a surnatant were obtained. Analysis for iron, carried out on the surnatant, only revealed Fe traces (less than 0.05% of total iron present in the reaction).

C) Isolation of the succinylated iron-polypeptide.

The above brown precipitate was recovered by decantation (or filtration or centrifugation) and washed with water at pH 3 (HCl); the precipitate was suspended in 5 ml of water and dissolved by addition of 1N NaOH until pH values were steadily higher than 7. The iron derivative was precipitated by acidification (addition of HCl to pH 3) and recovered by filtration; then it was redissolved in water by addition of NaOH to pH 7-7.2 and liophylized.

The obtained product was a succinylated polypeptide derivative containing 10.2% Fe w/w, as determined by reaction with O-phenanthroline, as described in Arzneim. Forsch. 34, (II), 9, 948-952, 1984. The derivative was further subjected to electrophoretic analysis (in order to verify the absence of bands attributable to the succinylated protein), ESR analysis to evaluate the valence status of iron in the complex and evaluation of succinylation degree.

ELECTROPHORESIS.

The analysis was carried out by cellulose acetate strips (previously treated with about 5 M Tris-trycine buffer pH 8.6 immediately before the sample deposition). A constant voltage of 40 V/cm for 30 minutes was applied to the strips, and the electrophoretic run was carried out using tris-tricine (pH 8.6) as the buffer. The electrophoretic bands were evidenced on the strip by staining with Ponceau red. The results found for the example sample showed no bands due to the succinylated protein present in FeSP and an electrophoretic band with mobility higher than that of FeSP.

ESR.

ESR spectra at 9,083 G of aqueous solutions of the derivative evidenced the presence of a band centered at g = 2.004, according to structures containing trivalent Fe atoms coupled by means of oxygen atoms.

SUCCINIC ACID CONTENT.

It was determined by enzymatic reaction with succinate thiokinase (Method of Enzymatic Analysis, Vol. 3, pp. 1616-1621, U. Bergmeyer Ed., Verlag Chemie, 1974) of the succinic acid released by the succinylated iron-polypeptide sample (100 mg), deprived of iron by treatment with 1N HCl and subjected to hydrolysis with 6N HCl (10 ml) for 12 hours at 100$^\bullet$C, under nitrogen atmosphere. The reaction revealed still the presence of succinic acid in the sample (in a content corresponding to 7.5% w/w).

EXAMPLE 2

A sample of FeSP (1000 mg), prepared as described in Example 1 (Fe content; 7.6% w/w) was dissolved in 50 ml of water and pH was adjusted to 7.8 by addition of NaOH. To the solution, respectively trypsin and chymotrypsin, in admixture, were added, in a 1:100 ratio with respect to the substrate, (specific enzyme activities 5000 U BAEE/ mg trypsin and 4000 U ATEE/mg chymotrypsin), or in a 0.01:100 and 10:100 ratio, (1:100 ratio being preferred). The reaction was stopped after about 2 hours, to obtain a succinylated polypeptide derivative containing 15.4% of iron, w/w, which can be easily dissolved in aqueous medium, at pH higher than 6.5.

EXAMPLE 3

A FeSP sample (2000 mg) containing 5% of iron was dissolved in 200 ml of NaOH (final solution pH = 7) and treated as described in Example 2.

A fraction was withdrawn at different times from the reaction medium and hydrolysis was interrupted by acidification (to pH about 3, by addition of HCl). The precipitate, containing iron, was then purified by re-dissolution in aqueous medium, as described in step C) of Example 1.

Succinylated polypeptide samples, containing iron amounts as reported in the following table, could be obtained:

TABLE I

| Fraction | Reaction time (min.) | Fe | Relative viscosity* compared with the solution at t = 0 |
|----------|----------------------|-----|--------------------------------------------------------|
| 1 | 15 | 6 | - 0.8 - |
| 2 | 60 | 8 | |
| 3 | 120 | 11 | - 0.1 - |
| 4 | 150 | 13 | |
| 5 | 500 | 20 | ---- |

* Expressed as the ratio of percolation time (Ostwald viscosimeter) of the sample solution to the one of FeSP solution, both of them being at the concentration (100 mg/10 ml).

It can clearly be seen that polypeptide derivatives containing desired iron amounts can be obtained from a FeSP sample containing a defined iron amount, by varying the protease reaction time.

EXAMPLE 4

Succinylated iron-polypeptide derivatives can be obtained as described in Examples 1 and 2, but using trypsin and chymotrypsin blocked on solid matrices which can be recovered and recycled. Particularly, FeSP solution (5000 mg of derivative dissolved in 250 ml o water) was brought to pH 8 and recycled on a cylinder reactor containing 20 ml of Sepharose gel, on which trypsin and chymotrypsin had been blocked (specific activities of blocked trypsin 4000 U BAEE/100 mg of Sepharose and chymotrypsin 2000 U ATEE /100 mg of Sepharose, respectively). The solution was recycled until viscosity was reduced to 1/10 of the starting one. Upon acidification to pH 3, succinylated iron-polypeptide was recovered, which was purified as described in Example 1C). The obtained derivative contained 19.5% of iron.

EXAMPLE 5

The procedure followed in Example 2 was repeated, but using pancreatin (as an example of the other proteases) instead of trypsin-chymotrypsin, in a 5:100 enzyme to substrate ratio.

A succinylated iron-polypeptide containing 14% w/w of Fe was obtained.

EXAMPLE 6

A FeSP sample (1000 mg) (prepared as described above, but with repeated $FeCl_3$ additions and readjusting pH to about 7 after each addition, to yield a derivative containing 20% of Fe) was vigorously stirred with 20 ml of water and added with NaOH till the final pH = 7.8 was constant. A gelly suspension was obtained which was treated for 24 hours with a trypsin-chymotrypsin mixture (enzyme to substrate ratio 3:100), as described in Example 2. The final clear solution was treated with HCl and the precipitate was purified by redissolution at pH 7 and reprecipitation with HCl (to pH 3.2), again dissolution at pH 7 and lyophilization. Upon analysis, the derivative turned out to consist of succinylated polypeptides containing 36% Fe (w/w).

EXAMPLE 7

A succinylated albumin sample containing 5.6% Fe was treated as described in Example 2. A succinylated polypeptide complex was obtained which was recovered and purified as above described (step C of Example 1). Said complex was highly soluble at pH > 5 and contained 12% Fe.

EXAMPLE 8

A sample of an iron complex with succinylated albumin (15% Fe) was treated with a trypsin-chymotrypsin mixture, as described in Example 4. A succinylated iron-polypeptide complex was recovered containing 25% Fe, highly soluble in water at pH > 7.

EXAMPLE 9

A casein sample was succinylated as described in Biochim. Biophys. Acta, 29, 587, 1958. The derivative was hydrolyzed with trypsin and chymotrypsin (enzyme to substrate ratio 1:0.2:100) for 60 minutes at about 30° C. The reaction pH was 7-8 and it was maintained constant by continous addition of NaOH. When the reaction was completed, pH was adjusted to 3.2 and a precipitate (about 40% of the starting material) was recovered which upon amino acid analysis showed a decrease in basic and neutral amino acids.

The polypeptide derivative, dissolved in NaOH (final pH = 7) at a concentration of 1 g/10 ml, was reacted with a $FeCl_3$ solution (1.5 g per gram of polypeptide derivative), keeping pH constant (7.8) during the addition of the iron solution. By acidification to pH 3.2 a precipitate was formed which, purified from unreacted iron by dissolution to pH 8 and reprecipitation with HCl, gave a polypeptide derivative containing 18.2% Fe (w/w), which was highly soluble in water at pH > 6.5.

EXAMPLE 10

3000 mg of solid casein (acid casein for alimentary use) were dissolved by addition of NaOH in water to a final volume of 50 ml. Trypsin (enzyme to substrate ratio 1:100) was added to the solution and the mixture was stirred for 3 hours. The hydrolytic reaction was stopped by acidification to pH 3.4 and the insoluble residue was recovered by centrifugation. The residue was dissolved in alkali medium (addition of 1N NaOH) to final pH = 7.6. Succinic anhydride (2 mg) was added portionwise, keeping pH at 7.6 by addition of 2N NaOH. The reaction was left to proceed at room temperature for about 2 hours. The formed succinylpeptides were recovered from the reaction medium by acidification (pH 3.4) with 2N HCl and centrifugation and purified from the succinic acid excess by repeated dissolutions (at pH 8) and precipitations (at pH 3.4) and centrifugations (removing each time the surnatant). The recovered succinylpeptides were redissolved in water by addition of NaOH (to a constant final pH of 7.6). A $FeCl_3$ solution was added to the obtained solution (in a ratio of 1.5 g each gram of the polypeptide derivative), keeping pH constant (7.6) during the addition. By acidification, a precipitate containing iron was formed, which was purified as described in the above example, to give a succinylated polypeptide derivative containing 17.5% Fe, which was highly soluble in water at pH > 6.5.

EXAMPLE 11

500 mg of polylysine (MW 17.000 D) were succinylated by a procedure analogous to that described in the above example, but using a polylysine: succinic anhydride ratio 1: 2 (w/w). Succinylated polylysine was purified by dialysis against distilled water and liophylized. The derivative obtained by succinylation (having a succinylation degree higher than 80%) was reacted with a $FeCl_3$ aqueous solution (25 mg/ml of solution). A precipitate was obtained which was freed from the iron ion excess and the salts by diafiltration, to give a final product containing 18% Fe (w/w) highly soluble in water.

EXAMPLE 12

The procedure of example 2 was repeated, but using an iron complex with succinylated soy proteins (Fe 9%). A polypeptide derivative containing 16% Fe was obtained, which was more soluble in water than the starting protein derivative.

## ORAL ABSORPTION OF SUCCINYLATED IRON POLYPEPTIDE

The iron absorption after oral administration of a succinylated iron-polypeptide sample was evaluated in the rat, by administering the derivative in aqueous solution, at a dose equivalent to 1 mg/kg of elemental iron and measuring the iron serum level against time. The results in Table II show the iron values found respectively in the animals treated with a derivative prepared as described in example 1 (column B), in the control animals (no administration, column A), and in case of administration of the starting FeSP (5.2% Fe, before hydrolysis, column C).

TABLE II

| TIME | (A) | (B) | (C) |
|------|-----|-----|-----|
| (h) | ug/100 ml | | |
| 0.5 | 95 | 270 | 230 |
| 1 | 100 | 280 | 274 |
| 2 | 95 | 280 | 270 |
| 4 | 90 | 250 | 200 |
| 6 | 85 | 160 | 150 |

The above data prove that succinylated iron-polypeptide can apport iron into plasmatic cycle, after oral administration, with an efficacy higher than the corresponding FeSP. Moreover, it should be stressed that succinylated polypeptides according to the invention show, in comparison with FeSP, advantages as regard tolerability, solubility and viscosity of the already mentioned solutions.

The present invention also relates to all the industrially applicable aspects relating to the use of succinylated iron-polypeptides as agent for the martial therapy. Therefore, a substantial object of the present invention is provided by pharmaceutical composition containing, as the active ingredient, predetermined and therapeutically effective amounts of succinylated iron-polypeptides (or the salts thereof) together with possible conventional excipients. Examples of said composition comprise tablets, sugar-coated pills, dragees, capsule, syrups, vials, etc., containing iron-polypeptides (or the salts thereof) in amounts corresponding to 20-160 mg of Fe per unitary dose, which can be administered 1-2 times a day.

**Claims**

1. Iron complexes with succinylated polypeptides, insoluble in water at pH lower than about 6.5 and soluble in water at pH higher than about 6.5 and the salts thereof with alkali or alkali-earth metals or with pharmaceutically acceptable organic bases.

2. Iron complexes according to claim 1, containing 0.1 to 45% by weight of iron.

3. Iron complexes according to claim 2, containing 10 to 45% by weight of iron.

4. Pharmaceutical and veterinary compositions for the martial therapy, containing as active ingredients one or more complexes according to claims 1-3.

5. Pharmaceutical and veterinary compositions according to claim 4, containing 20 to 160 mg of iron bonded in complexes with succinylated polypeptides, per unitary dose.

6. A process for the preparation of the iron complexes with succinylated polypeptides according to claims 1-3, which process consists in subjecting iron complexes with succinylated proteins to enzymatic degradation with one or more proteolytic enzymes, at pH kept constant from 6 to 10 during the reaction, and at temperatures from 0° to 50° C, till attainment of succinylpolypeptides complexes having the desired iron content, and in recovering the latter by diafiltration and lyophilization, or by acidification of the reaction mixture (previously filtered from any insoluble) at pH 2-3.5, the obtained precipi tate possibly being redissolved in aqueous medium at neutral or basic pH and finally isolated from the aqueous solution by per se known methods.

7. A process according to claim 6, which is carried out at pH from 7.5 to 8.5 and at temperatures from 20 to 30° C.

8. A process according to claims 6 and 7, which is carried out using enzymes selected from the group consisting of trypsin, chymotrypsin and pancreatine.

9. A process according to claims 6-8, which is carried out in an enzyme: substrate weight ratio of about 1:100.

10. A process for the preparation of iron complexes with succinylated polypeptides according to claims 1-3, in which process iron ions are reacted with succinylated polypeptides at pH from 7 to 8, and the obtained complexes are isolated according to claim 6.

11. A process according to claim 10, in which succinylated polypeptides from enzymatic degradation of succinylated proteins are used.

12. A process according to claim 10, in which succinylated polypeptides from the succinylation of polypeptides are used.

Claims for the following Contracting States: ES, GR.

1. A process for the preparation of the iron complexes with succinylated polypeptides insoluble in water at pH lower than about 6.5 and soluble in water at pH higher than about 6.5, which process consists in subjecting iron complexes with succinylated proteins to enzymatic degradation with one or more proteolytic enzymes, at pH kept constant from 6 to 10 during the reaction, and at temperatures from 0° to 50° C, till attainment of succinylpolypeptides complexes having the desired iron content, and in recovering the latter by diafiltration and lyophilization, or by acidification of the reaction mixture (previously filtered from any insoluble) at pH 2-3.5, the obtained precipitate possibly being redissolved in aqueous medium at neutral or basic pH and finally isolated from the aqueous solution by per se known methods.

2. A process according to claim 1, which is carried out at pH from 7.5 to 8.5 and at temperatures from 20 to 30° C.

3. A process according to claims 1 and 2, which is carried out using enzymes selected from the group consisting of trypsin, chymotrypsin and pancreatine.

4. A process according to claims 1-3, which is carried out in an enzyme: substrate weight ratio of about 1:100.

5. A process for the preparation of iron complexes with succinylated polypeptides according to claim 1, in which process iron ions are reacted with succinylated polypeptides at pH from 7 to 8, and the obtained complexes are isolated according to claim 6.

6. A process according to claim 5, in which succinylated polypeptides from enzymatic degradation of succinylated proteins are used.

7. A process according to claim 5, in which succinylated polypeptides from the succinylation of polypeptides are used.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | GB-A-2 115 821  (ITALFARMACO S.p.A.)<br>* Whole document * | 1-12 | A 61 K  37/14<br>A 61 K  37/18<br>C 12 P  21/06 |
| Y | US-A-4 172 072  (ASHMEAD)<br>* Whole document * | 1-12 | |
| A | US-A-3 969 540  (JENSEN)<br>* Whole document * | 1-12 | |
| A | US-A-4 208 405  (FOUAD)<br>* Column 6, lines 1-59 * | 1-12 | |
| A | GB-A-  673 063  (MEDICAL RESEARCH PROPRIETARY LTD)<br>* Claims * | 1-12 | |
| A | US-A-4 216 144  (ASHMEAD) | | |
| A | US-A-4 167 564  (JENSEN) | | |
| P,X | EP-A-0 243 322  (MAGI FARMACEUTICI)<br>* Whole document * | 1-12 | TECHNICAL FIELDS SEARCHED (Int. Cl.4)<br><br>A 61 K<br>C 12 P |
| A | BIOLOGICAL ABSTRACTS, vol. 79, 1985, no. 97946, Biological Abstracts, Inc., Philadelphia, PA, US; M.I. SCHNEPF et al.: "Partial characterization of an iron soy protein complex", & NUTR. REP. INT. 31(2): 371-380, 1985 | | |
| A | JOURNAL OF FOOD SCIENCE, vol. 45, 1980, pages 1200-1201, Institute of Food Technologists; F.C. WOODS et al.: "Protease from saccharomyces carlsbergensis: activity on food proteins"Page 1200, column 1; page 1201, column 2, lines 4-30 | | |

The present search report has been drawn up for all claims

| Place of search | Date of completion of the search | Examiner |
|---|---|---|
| THE HAGUE | 27-01-1989 | SKELLY J.M. |

EPO FORM 1503 03.82 (P0401)